# EUROPEAN PATENT APPLICATION

(11) **EP 1 348 413 A1**
(43) Date of publication of application: **01.10.2003**
(21) Application number: 03251710.4
(22) Date of filing: 19.03.2003
(51) Int. Cl.: A61F 13/15

(54) **Absorbant article and manufacturing method thereof**

(30) Priority: 29.03.2002 JP 2002094890
(71) Applicant: UNI-CHARM CORPORATION, Kawanoe-shi Ehime-ken (JP)
(72) Inventor: Kudo, Jun, c/o Technical Center, Mitoyo-gun, Kagawa-ken 769-1602 (JP); Suekane, Makoto, c/o Technical Center, Mitoyo-gun, Kagawa-ken 769-1602 (JP)
(74) Representative: Parry, Christopher Stephen

(57) **Abstract**

Disclosed is an absorbent article including a liquid-permeable top layer, a backsheet, and an absorbent layer disposed between the top layer and the backsheet. Fibers constituting the absorbent layer are bonded together. The absorbent layer includes hydrophilic fibers. The top layer and the absorbent layer are embossed together to have recesses in which fibers constituting the absorbent layer are bonded to the top layer.

## Description

### BACKGROUND OF THE INVENTION

### Field of the Invention

The present invention relates to a thin absorbent article such as vaginal discharge absorbing sheet, generally called panty liner, more particularly, relates to an absorbent article having a pleasant feel in which highly viscous body fluid can be readily drawn into an absorbent layer. The present invention also relates to a method for manufacturing the absorbent article.

### Description of the Related Art

Vaginal discharge absorbing sheet, generally called panty liner, has been known as thin absorbent article. Such absorbing sheet is to be worn while being fixed to an inner side of a crotch portion of an undergarment through a pressure sensitive adhesive layer. This absorbent article is generally constructed such that an absorbent layer for absorbing liquid is disposed beneath a liquid-permeable top layer so that body fluid given to the top layer can be drawn in by capillary action of the absorbent layer.

However, because highly viscous body fluid secreted from the vagina is given to such vaginal discharge absorbing sheet, if the top layer is thick, the body fluid given to the top layer cannot be readily drawn in by capillary action of the absorbent layer, resulting in that liquid absorption capacity cannot be sufficiently displayed.

In order to solve this problem, there have been developed a vaginal discharge absorbing sheet in which the top layer itself can display liquid absorption capacity and a vaginal discharge absorbing sheet in which an extremely thin liquid-permeable top layer is disposed on the absorbent layer. In these cases, however, body fluid tends to be left in the top layer, so that wearers often feel uncomfortable.

In the field of sanitary napkins that are thicker than the vaginal discharge absorbing sheets, on the other hand, Japanese Unexamined Patent Publication No. 8-71105 (71105/1996) discloses a sanitary napkin in which an absorbent layer comprising pulp or pulp and synthetic fibers is disposed beneath a liquid-permeable nonwoven sheet as top layer and embossing is performed to have a plurality of recesses from the top layer to the absorbent layer. In this sanitary napkin, body fluid given to the liquid-permeable top layer can be readily absorbed by the absorbent layer through the recesses.

However, although the absorbent layer disclosed in the above-identified Patent Publication comprises fluff pulp or a mixture of fluff pulp and synthetic fibers, the absorbent layer is not constructed on the basis of the technical idea of increasing wet strength. Accordingly, the recesses tend to disappear when a pressure is applied from the wearer's body to the sanitary napkin in a wet state. However, the absorbent article disclosed in the above-identified Patent Publication relates to a sanitary napkin. Since menstrual blood is not highly viscous as compared with vaginal discharge, it can be readily drawn into the absorbent layer via the top layer by capillary action of the absorbent layer. Therefore, the possibility of disappearance of the recesses is relatively permissible.

However, if the vaginal discharge absorbing sheet as thin absorbent article is constructed in the same manner as disclosed in the above-identified Patent Publication, i.e., the thin absorbent layer of the vaginal discharge absorbing sheet is embossed together with the top layer to have recesses from the top layer to the absorbent layer, the recesses become too shallow. As a result, the recesses easily disappear in a wet state when a pressure is applied from the wearer's body, as compared with the sanitary napkin. Moreover, since vaginal discharge secreted from the vagina is more viscous than menstrual blood, if the recesses disappear, such highly viscous body fluid cannot be readily drawn into the absorbent layer via the top layer.

### SUMMARY OF THE INVENTION

The present invention has been worked out in view of the shortcoming in the prior art set forth above. It is therefore an object of the present invention to provide a relatively thin absorbent article, in which recesses formed from a top layer to an absorbent layer hardly disappear by pressure so that relatively highly viscous body fluid given to the top layer can be certainly absorbed by the absorbent layer, and a manufacturing method thereof.

According to a first aspect of the present invention, there is provided an absorbent article comprising:
a liquid-permeable top layer;
a backsheet; and
an absorbent layer disposed between the top layer and the backsheet, fibers constituting the absorbent layer being bonded together, wherein
the absorbent layer comprises hydrophilic fibers, and
the top layer and the absorbent layer are embossed together to have recesses in which fibers constituting the absorbent layer are bonded to the top layer.

In the absorbent article of the present invention, since fibers constituting the absorbent layer are bonded together, the absorbent layer has an increased wet strength. Therefore, disappearance of the recesses due to a pressure applied from the body of a wearer during wear hardly occurs in a wet state, so that the recesses can be certainly maintained even when the absorbent layer is thin. Accordingly, body fluid can be easily absorbed by the absorbent layer through the recesses at all times.

In order to prevent the disappearance of the recesses, it is preferred that the absorbent layer has a wet tensile strength of at least 2 N per 25 mm width.

Preferably, the absorbent layer further comprises heat-fusible synthetic fibers, and the synthetic fibers are fusion-bonded to the hydrophilic fibers inside the absorbent layer. In an alternative, fibers constituting the absorbent layer may be bonded together with an adhesive. For instance, the absorbent layer may be air-laid nonwoven fabric.

Preferably, the top layer comprises a heat-fusible material and fibers constituting the absorbent layer are fusion-bonded to the top layer in the recesses. In an alternative, fibers constituting the absorbent layer may be bonded to the top layer in the recesses with an adhesive.

Preferably, the top layer comprises at least one sheet of nonwoven fabric and has a basis weight of 15 to 100 g/m² and a bulk of 0.2 to 1.5 mm. The basis weight is more preferably in the range of 20 to 40 g/m². With the top layer being made thus bulky, excellent touch can be obtained. Even in this case, since the recesses hardly disappear, relatively highly viscous body fluid such as vaginal discharge can be certainly introduced into the absorbent layer through the recesses.

Preferably, the top layer comprises two sheets of nonwoven fabric, and the top layer and the absorbent layer are bonded to each other with an adhesive that is applied in a pattern different from that of an adhesive applied for bonding the two sheets of nonwoven fabric to each other. In the case where the adhesive application pattern between the two sheets of nonwoven fabric and the adhesive application pattern between the top layer and the absorbent layer are different from each other, the absorbent article can provide a soft feel.

Preferably, the absorbent layer is of an elongated shape, and the top layer and the backsheet are bonded to each other outside the absorbent layer to have side regions extending longitudinally of the absorbent layer. In this case, since the absorbent layer is present in a region for contacting the vagina of a wearer but is absent from the side regions, the side regions can deform freely, so that the absorbent article can be worn comfortably.

Accordingly, the absorbent article of the present invention is suitable for use as a vaginal discharge absorbing sheet.

According to a second aspect of the present invention, there is provided a method for manufacturing an absorbent article having a liquid-permeable top layer, a backsheet, and an absorbent layer disposed between the top layer and the backsheet, the method comprising:
letting out an absorbent layer from a material roll, wherein fibers constituting the absorbent layer are bonded together and the absorbent layer comprises hydrophilic fibers, and heating the absorbent layer to restore bulk;
bonding a top layer to the absorbent layer;
embossing the top layer and the absorbent layer together to have recesses;
laying a backsheet on a garment surface of the absorbent layer; and
cutting the top layer and the backsheet into the shape of an absorbent article.

In the absorbent article manufacturing method, since the absorbent layer in which fibers are bonded together to increase a wet strength is heated to restore bulk, the density of the absorbent layer can be set within a preferred range, so that liquid absorption capacity and liquid retention capacity of the absorbent layer can be increased as well as liquid can be prevented from flowing back.

Preferably, the absorbent article manufacturing method further comprises heating the top layer to restore bulk before bonding the top layer to the absorbent layer. The top layer can be made bulky by heating the top layer to restore bulk, so that the absorbent article can be worn comfortably.

### BRIEF DESCRIPTION OF THE DRAWINGS

The present invention will be understood more fully from the detailed description given hereinafter and from the accompanying drawings of the preferred embodiment of the present invention, which, however, should not be taken to be limitative to the invention, but are for explanation and understanding only.

In the drawings:
Fig. 1 is a partially cutaway perspective view showing a vaginal discharge absorbing sheet as an absorbent article according to a first embodiment of the present invention;
Fig. 2 is a sectional view taken along line II-II of Fig. 1;
Fig. 3 is an enlarged view of a portion of Fig. 2;
Fig. 4 is a top plan view of the absorbing sheet;
Figs. 5A, 5B and 5c illustrate application patterns of a hot-melt type adhesive;
Fig. 6 is a partially enlarged sectional view showing a vaginal discharge absorbing sheet as an absorbent article according to a second embodiment of the present invention; and
Fig. 7 illustrates an absorbent article manufacturing method according to one embodiment of the present invention.

### DESCRIPTION OF THE PREFERRED EMBODIMENT

The present invention will be discussed hereinafter in detail in terms of the preferred embodiment according to the present invention with reference to the accompanying drawings. In the following description, numerous specific details are set forth in order to provide a thorough understanding of the present invention. It will be obvious, however, to those skilled in the art that the present invention may be practiced without these specific details. In other instance, well-known structures are not shown in detail in order to avoid unnecessary obscurity of the present invention.

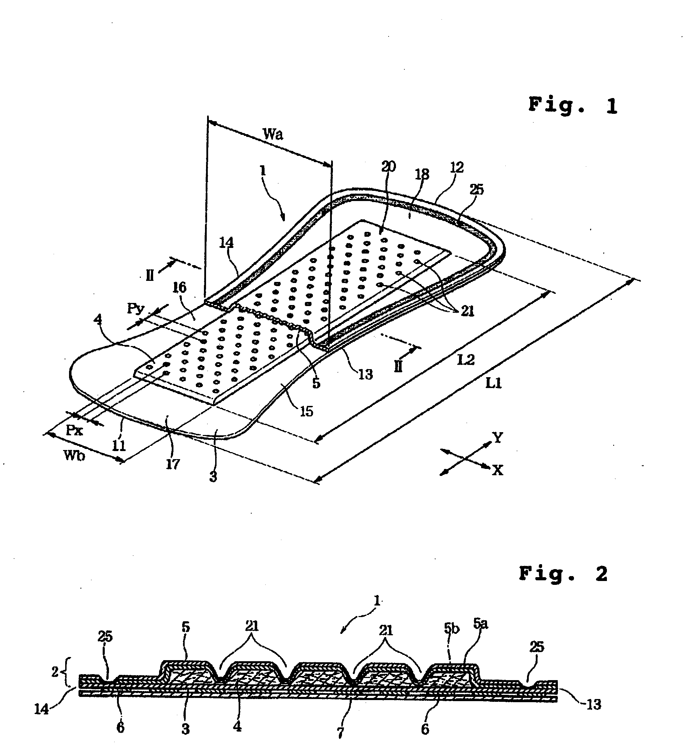

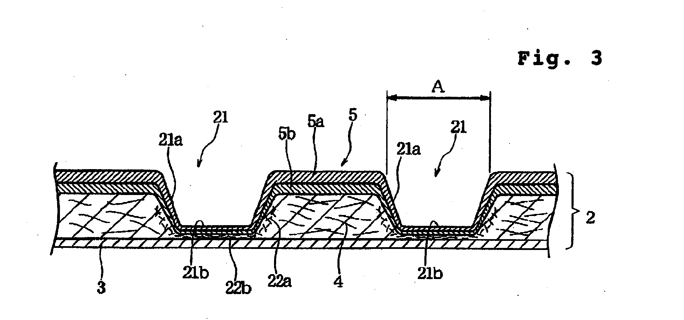

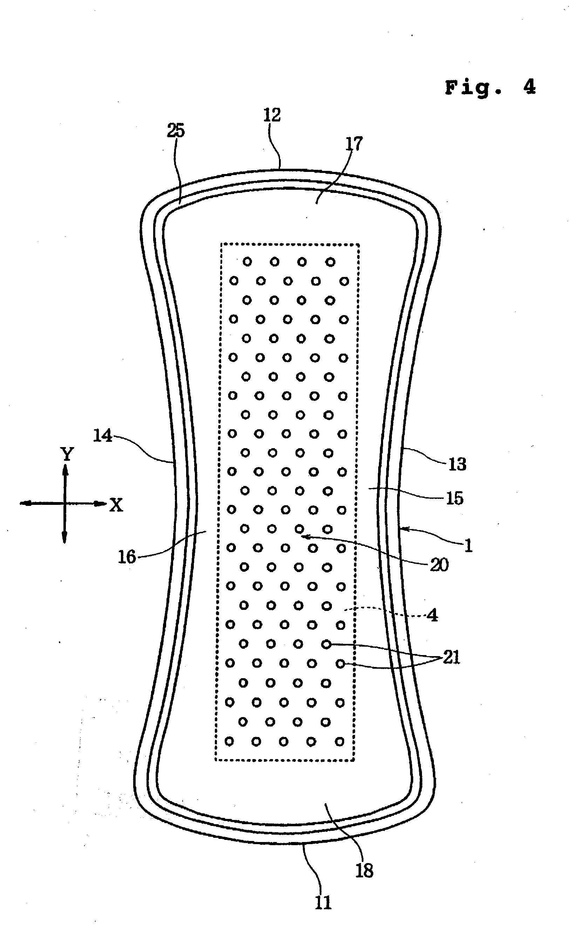

Fig. 1 is a partially cutaway perspective view showing a vaginal discharge absorbing sheet, generally called panty liner, as a thin absorbent article according to a first embodiment of the present invention; Fig. 2 is a sectional view taken along line II-II of Fig. 1; Fig. 3 is an enlarged view of a portion of Fig. 2; and Fig. 4 is a top plan view of the absorbing sheet.

The vaginal discharge absorbing sheet designated generally by 1 is a thin absorbent article that is to be used as an integral part of an undergarment while being fixed to an inner side of a crotch portion of the undergarment. This absorbing sheet 1 is intended to absorb vaginal discharge and so on discharged from women. It should be noted that individual components of the absorbing sheet 1 have a body surface and a garment surface. As used herein, the " body surface" means that surface of the components which is intended to be worn toward or adjacent to the body of a wearer, while the " garment surface" is on the opposite side and is intended to be worn toward or placed adjacent to the undergarment when the absorbing sheet 1 is worn.

As shown in Fig. 1, the absorbing sheet 1 is of an hourglass shape, wherein a front edge 11 and a rear edge 12 that are intended to face the abdomen and the buttocks of a wearer are outwardly curved and longitudinally extending side edges 13 and 14 are inwardly curved. In Figures, Y-direction extending from the front edge 11 to the rear edge 12 represents a longitudinal direction of the absorbing sheet 1; and X-direction extending from the side edge 13 to the side edge 14 represents a transverse direction of the absorbing sheet 1.

As shown in Fig. 2, the absorbing sheet 1 has a main body portion 2 that is a stack of a backsheet 3, an absorbent layer 4 laid on the backsheet 3, and a top layer 5 laid on the absorbent layer 4. As shown in Fig. 3 at an enlarged scale, the top layer 5 comprises first nonwoven fabric 5a appearing externally and second nonwoven fabric 5b beneath it.

As shown in Fig. 2, a pressure sensitive adhesive layer 6 for fixing the main body portion 2 to the undergarment is provided on the garment surface of the main body portion 2, i.e., the garment surface of the backsheet 3. This pressure sensitive adhesive layer 6 is applied in the shape of strips that are spaced apart from each other in the transverse direction (X-direction) and individually extend linearly in the longitudinal direction (Y-direction). The garment surface of the pressure sensitive adhesive layer 6 is covered with a release sheet 7.

As viewed from above, the backsheet 3, the top layer 5 and the release sheet 7 are of the same shape and size. That is, all the backsheet 3, the top layer 5 and the release sheet 7 are of the hourglass shape having the front edge 11, the rear edge 12 and the side edges 13 and 14.

The absorbent layer 4 is elongated in the longitudinal direction to have a rectangular shape. A width Wb of the absorbent layer 4 is smaller than a minimum width Wa of the absorbing sheet 1; and a length L2 of the absorbent layer 4 is smaller than a length L1 of the absorbing sheet 1. Outside the absorbent layer 4, the backsheet 3 and the top layer 5 overlap with each other to have left and right side regions 15 and 16 and front and rear end regions 17 and 18. The region having the absorbent layer 4 is referred to as liquid absorbing region 20.

In the absorbing sheet 1, the liquid absorbing region 20 that is located centrally as viewed from above is intended to mainly contact the vagina of a wearer. The left and right side regions 15 and 16 and the front and rear end regions 17 and 18 are soft regions that can be easily deformed due to absence of the absorbent layer 4.

In the top layer 5, the first nonwoven fabric 5a and the second nonwoven fabric 5b are bonded to each other with a hot-melt type adhesive. The second nonwoven fabric 5b of the top layer 5 is also bonded to the absorbent layer 4 with a hot-melt type adhesive. In this embodiment, the application pattern of the adhesive for bonding the first nonwoven fabric 5a to the second nonwoven fabric 5b is different from that of the adhesive for bonding the second nonwoven fabric 5b to the absorbent layer 4.

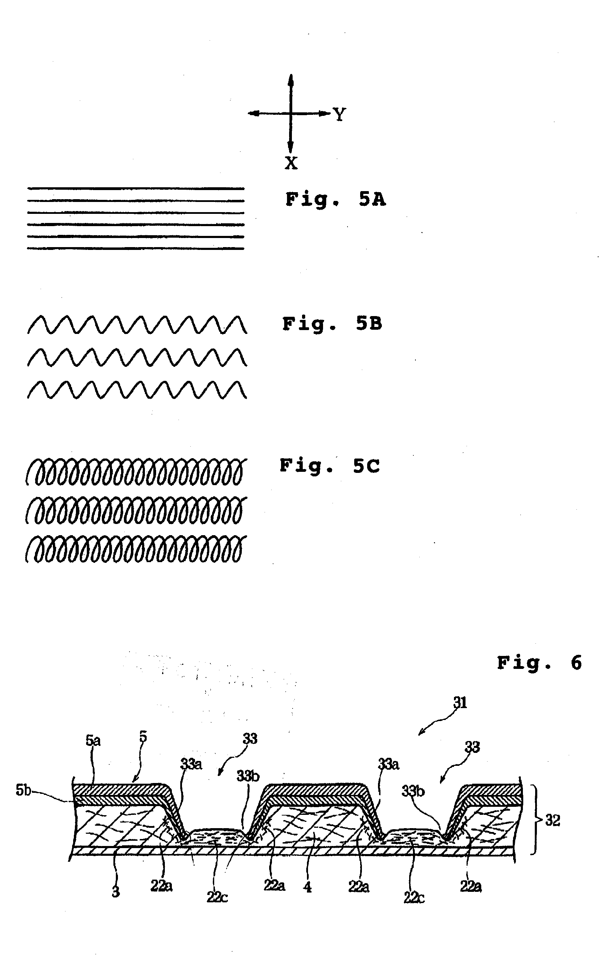

For instance, one adhesive application pattern is a pattern of parallel straight lines or strips that are spaced apart from each other in the transverse direction and individually extend in the longitudinal direction (see Fig. 5A); the other adhesive application pattern is a pattern of parallel wavy lines that individually oscillate in the transverse direction to have arcuate portions repeated in the longitudinal direction (see Fig. 5B) or a pattern of parallel spiral lines that individually extend in the longitudinal direction (see Fig. 5C).

In the case where the adhesive application patterns are different from each other, a total area of overlapping portions between the adhesive applied between the first nonwoven fabric 5a and the second nonwoven fabric 5b and the adhesive applied between the second nonwoven fabric 5b and the absorbent layer 4 can be minimized, so that the stiffness due to the presence of the adhesive can be reduced in the boundary between the top layer 5 and the absorbent layer 4 that have a great influence on the touch. Therefore, the touch can be improved.

After the absorbent layer 4 and the top layer 5 are bonded to each other, moreover, the absorbent layer 4 and the top layer 5 are recessed together to have a large number of recesses 21 for introducing liquid, as shown in Fig. 3. As shown in Fig. 1, these recesses 21 are distributed exclusively over the liquid absorbing region 20 in a staggered manner.

Each recess 21 has a generally conical (tapered) wall portion 21a that is narrowed toward the absorbent layer 4. In the embodiment of Fig. 3, the first nonwoven fabric 5a and the second nonwoven fabric 5b constituting the top layer 5 are present in both the wall portion 21a and a bottom portion 21b. As will be described later, both the first nonwoven fabric 5a and the second nonwoven fabric 5b of the top layer 5 are heat-fusible, and the absorbent layer 4 contains thermoplastic resin fibers that are also heat-fusible.

The recesses 21 can be formed such that after the absorbent layer 4 and the top layer 5 are bonded to each other, the absorbent layer 4 and the top layer 5 are compressed by forcing heated pins having the shape of a cut-off cone against the top layer 5 toward the absorbent layer 4. At this time, fibers constituting the top layer 5 and the thermoplastic resin fibers contained in the absorbent layer 4 are fusion-bonded to each other. As a result, fused regions 22a in which fiber density is increased are formed along boundaries between the wall portions 21a and the absorbent layer 4, and fused regions 22b in which fiber density is increased are formed along boundaries between the bottom portions 21b and the absorbent layer 4.

After the formation of the recesses 21, the backsheet 3 that is liquid-impermeable is laid on the garment surface of the absorbent layer 4. At this time, a hot-melt type adhesive is applied on the body surface of the liquid-impermeable backsheet 3. Accordingly, the backsheet 3 is bonded not only to the garment surface of the absorbent layer 4 but also to the second nonwoven fabric 5b of the top layer 5 in the left and right side regions 15 and 16 and the front and rear end regions 17 and 18.

It should be noted that a round seal 25 of a predetermined width is formed along the entire periphery of the main body portion 2 while leaving peripheral regions of a predetermined width inside the individual edges 11, 12, 13 and 14, as shown in Fig. 1. This round seal 25 is a set of dotted small fused regions, wherein the first nonwoven fabric 5a and the second nonwoven fabric 5b constituting the top layer 5 are fusion-bonded to each other under pressure.

The absorbent layer 4 preferably comprises a mixture of hydrophilic fibers and thermoplastic resin fibers as heat-fusible synthetic fibers, wherein at least surface of the thermoplastic resin fibers are melted under heat so that the hydrophilic fibers are bonded to the surface of the thermoplastic resin fibers. In this absorbent layer 4, since the hydrophilic fibers are bonded to the thermoplastic resin fibers, an internal structure can be certainly maintained. The absorbent layer 4 has such an internal structure that liquid drawn into the absorbent layer 4 can be easily retained in voids (cells) between fibers due to wettability of the hydrophilic fibers and capillary action between fibers. In case where the absorbent layer 4 contains 5 to 30 % by weight of thermoplastic resin fibers having a fineness of 1.5 to 3.3 dtex and a length of 5 to 20 mm, wet strength of the absorbent layer 4 can be sufficiently increased.

Accordingly, the absorbent layer 4 can exhibit excellent resiliency under pressure, so that the voids are hardly eliminated. Therefore, liquid once retained by the absorbent layer 4 hardly flows back to the top layer 5. In addition, since the strength of the absorbent layer 4 is sufficiently increased in the portion where the recesses 21 are not formed, the disappearance of the recesses 21 hardly occurs even when a pressure is given to the absorbent layer 4. Therefore, the shape of the recesses 21 for introducing liquid can be certainly maintained at all times.

In order to provide such a high wet tensile strength and such voids for retaining liquid, the absorbent layer 4 is preferably formed of air-laid nonwoven fabric.

The air-laid nonwoven fabric can be manufactured such that a mixture of the hydrophilic fibers and the thermoplastic resin fibers having been floated in air and deposited onto a screen is heated to melt the surface of the thermoplastic resin fibers so that the thermoplastic resin fibers are fusion-bonded to the hydrophilic fibers.

For instance, the air-laid nonwoven fabric comprises: 87 % by weight of pulp as the hydrophilic fibers; and 13 % by weight of sheath/core bicomponent fibers (a fineness of 1.7 dtex, a length of 13 mm) of which the core component is made of PP (polypropylene) and the sheath component is made of PE (polyethylene).

For the hydrophilic fibers, use can be made of one kind of fibers selected from wood pulp, rayon, acetate rayon, natural cellulose fibers other than pulp, mercerized pulp and crosslinked pulp or a mixture of two or more kinds thereof. For the heat-fusible thermoplastic resin fibers, use can be made of the PE/PP sheath/core bicomponent fibers, PE/PET (polyethylene terephthalate) sheath/core bicomponent fibers or monocomponent fibers of PE, PP or PET.

Fibers may be bonded together by spraying an emulsion type adhesive in the production process of the air-laid nonwoven fabric. It is also possible that the air-laid nonwoven fabric contains superabsorbent polymer.

The air-laid nonwoven fabric is dried, wound into a material roll, and then, supplied in the production process of the absorbing sheet 1. In this production process, the air-laid nonwoven fabric let out from the material roll is heated at a temperature of 100 °C or more by using a heating roller or applying heated air to restore bulk (thickness) in the range of 5 to 20%, and immediately thereafter, cooled by air. The bulk of the air-laid nonwoven fabric is reduced due to pressure when it is wound into the material roll, but the bulk can be restored by such heating treatment.

In order that the shape of the recesses 21 can be certainly maintained and the voids are hardly eliminated in the absorbent layer 4, the absorbent layer 4 preferably has a wet tensile strength of at least 2 N per 25 mm width. Here, the upper limit of the wet tensile strength need not be specifically defined, but if the wet strength is extremely increased, a wearer may feel stiffness. Accordingly, the upper limit of the tensile strength is preferably at most 10 N, more preferably at most 8 N.

Also preferably, the absorbent layer 4 has a density of 0.035 to 0.13 g/cm³ so as to have suitable voids for retaining liquid in the absorbent layer 4. If the density is less than 0.035 g/cm³, the voids for retaining liquid in the absorbent layer 4 can be made large but liquid can move excessively freely in the voids, so that the liquid once retained by the absorbent layer 4 easily flows back to the top layer 5. If the density is greater than 0.13 g/cm³, on the other hand, liquid can be easily drawn in due to capillary action but liquid retention capacity will be lowered because the voids are too small.

The absorbent layer 4 may have a basis weight within the range of 40 to 300 g/m². In the vaginal discharge absorbing sheet 1, the absorbent layer 4 should be thin to have a thickness within the range of 0.35 to 3.5 mm.

The first nonwoven fabric 5a and second nonwoven fabric 5b constituting the top layer 5 are preferably bulky nonwoven fabric comprising heat-fusible thermoplastic resin fibers, such as through-air bonded nonwoven fabric in which the thermoplastic resin fibers are fusion-bonded together with heated air. For instance, the through-air bonded nonwoven fabric may be formed of a mixture of: 10 to 60% by weight of PE/PP sheath/core bicomponent fibers that are whitened by adding titanium oxide to the PP core component in an amount of 3 to 5% by weight of fiber; and 90 to 40% by weight of PE/PET sheath/core bicomponent fibers that are whitened by adding titanium oxide to the PET core component in an amount of 0.3 to 2% by weight of fiber. These fibers used for forming the through-air bonded nonwoven fabric have a fineness in the range of 1.5 to 4.4 dtex and a length in the range of 20 to 60 mm. The through-air bonded nonwoven fabric using such fibers can be made resilient, so that the bulk can be certainly maintained even when a pressure is applied from the body of a wearer.

The through-air bonded nonwoven fabric is first wound into a material roll, and then, let out from the material roll. For both the first nonwoven fabric 5a and the second nonwoven fabric 5b, the through-air bonded nonwoven fabric let out from the material roll is heated at a temperature of about 115 °C by using a heating roller or applying heated air to restore bulk (thickness) in the range of 1.5 to 8 times, and then, rapidly cooled. The top layer 5 composed of two sheets of the nonwoven fabric may have a basis weight within the range of 15 to 100 g/m², preferably within the range of 20 to 40 g/m². The top layer 5 preferably has a bulk (thickness) of 0.2 to 1.5 mm. Also in the case where the top layer 5 is composed of a single sheet of the through-air bonded nonwoven fabric, the top layer 5 preferably has a basis weight and a bulk within the above-mentioned ranges. The top layer 5 preferably has a fiber density of 0.01 to 0.02 g/cm³.

The backsheet 3 is a liquid-impermeable sheet, such as thin polyethylene film having a basis weight of 10 to 50 g/m², that is preferably provided with pores so as to be moisture-permeable (breathable). In an alternative, the backsheet 3 may be hydrophobic nonwoven fabric.

Since the top layer 5 composed of the through-air bonded nonwoven fabric is bulky and the recesses 21 are arranged in the staggered manner, the body surface of the absorbing sheet 1 can provide a soft feel, so that the touch can be improved. Here, since the top layer 5 is made so bulky, the body fluid discharging part (vagina) and the absorbent layer 4 are spaced apart from each other largely for the thickness of the top layer 5, but vaginal discharge that is relatively highly viscous can be directly introduced into the recesses 21 and then introduced into the absorbent layer 4 due to the capillary action of the fused regions 22a and 22b of a higher fiber density that are formed around the recesses 21. Thereafter, the vaginal discharge can be diffused in the surroundings of the recesses 21 due to the capillary action of the fibers constituting the absorbent layer 4 and retained in the voids.

As has been described hereinabove, since the strength of the top layer 4 is increased by fusion-bond between the heat-fusible thermoplastic resin fibers, the structure around the recesses 21 is strong enough to prevent the recesses 21 from disappearing when a pressure is applied from the body of a wearer. In the absorbing sheet 1, more specifically, the top layer 5 and the absorbent layer 4 are bonded to each other, the absorbent layer 4 is resilient enough to maintain its shape, and the fused regions 22a and 22b are formed around the recesses 21 to have an increased strength. Therefore, even after a shearing force acts on the surface of the top layer 5 in the transverse or longitudinal direction to close the recesses 21, the recesses 21 can immediately recover their shape. In addition, even after a pressure acts on the surface of the top layer 5 from above and the absorbent layer 4 is compressed, the recesses 21 can easily recover their shape.

Each recess 21 preferably has an opening area A in the range of 0.1 to 7 mm², as measured in a plane flush with the body surface of the unrecessed portion in the liquid absorbing region 20, and both the transverse pitch Px and the longitudinal pitch Py of the recesses 21 are preferably in the range of 0.5 to 10 mm. The ratio of the area occupied by the recesses 21 to the area of the liquid absorbing region 20 is preferably in the range of 3 to 30%. If less than 3%, body fluid cannot be efficiently introduced into the absorbent layer 4 through the recesses 21. If greater than 30%, on the other hand, the surface of the liquid absorbing region 20 cannot provide a soft feel.

The width Wb of the liquid absorbing region 20 is preferably in the range of 20 to 50 mm. If within this range, the liquid absorbing region 20 can be certainly brought into contact with the vagina of a wearer. The minimum width of the individual side regions 15 and 16, i.e., (Wa - Wb)/2 is preferably in the range of 5 to 15 mm. If within this range, the left and right side regions 15 and 16 outside the absorbent layer 4 can easily deform to conform to an undergarment when the absorbing sheet 1 is attached to an inner side of the undergarment and worn.

The length L2 of the liquid absorbing region 20 is preferably equal to or greater than 80 mm. If the length L2 is equal to or greater than 80 mm, the liquid absorbing region 20 can be certainly brought into contact with the vagina of a wearer, so that body fluid such as vaginal discharge can be certainly received in the liquid absorbing region 20. The liquid absorbing region 20 may extend over the whole of the absorbing sheet 1. Accordingly, the upper limit of the length L2 depends on the length L1 of the absorbing sheet 1. The length L1 is from 100 to 350 mm.

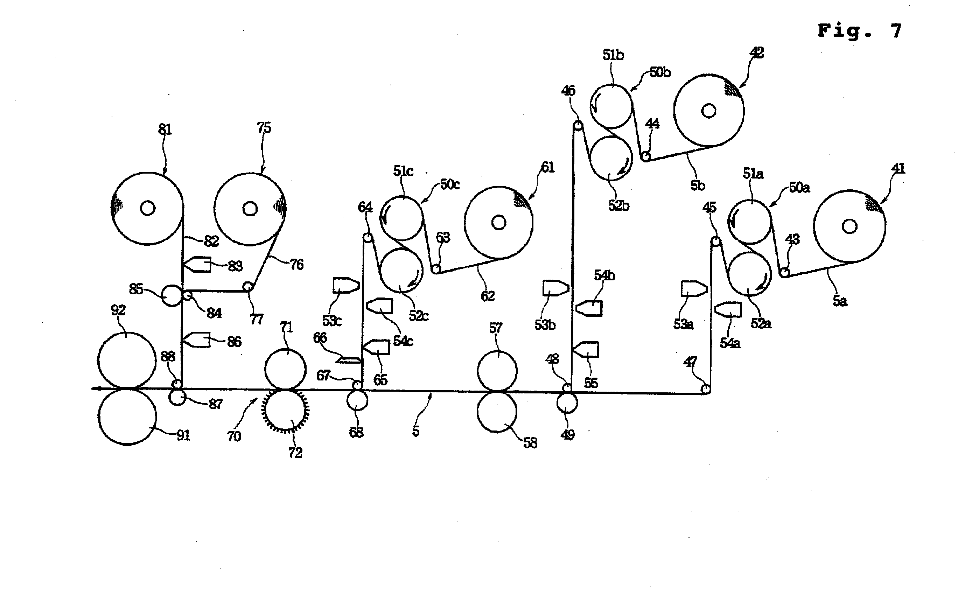

Fig. 6 is an enlarged sectional view showing a portion of a main body portion 32 of a vaginal discharge absorbing sheet 31 according to a second embodiment of the present invention. Here, the detailed description of the portions having the same constructions as those of the vaginal discharge absorbing sheet 1 according to the first embodiment will be omitted by designating them by the common reference numerals.

In the absorbing sheet 31 of Fig. 6, the main body portion 32 is recessed from the top layer 5 toward the absorbent layer 4 to have recesses 33. Each recess 33 has a tapered wall portion 33a that remains covered with the top layer 5, but the top layer 5 has an opening 33b at the bottom portion of each recess 33, so that the absorbent layer 4 is exposed externally in the opening 33b. Along the wall portion 33a, the absorbent layer 4 is compressed and the top layer 5 and the absorbent layer 4 are fusion-bonded to each other to form the fused region 22a in which the absorbent layer 4 has an increased density. When embossing is performed with heated pins, the top layer 5 is pressed and the openings 33b are simultaneously formed. Accordingly, the absorbent layer 4 is heated under pressure at the externally exposed portions in the openings 33b, thereby forming compressed portions 22c. Also in these compressed portions 22c, the absorbent layer 4 has an increased density.

In the absorbing sheet 31 of Fig. 6, liquid given to the recesses 33 can be drawn in due to the capillary action of the fused regions 22a and the compressed portions 22c, and then diffused in the absorbent layer 4.

When the absorbing sheet 1 of Fig. 3 and the absorbing sheet 31 of Fig. 6 are compared, the absorbing sheet 31 has an advantage in that body fluid can be rapidly absorbed because highly viscous body fluid, such as vaginal discharge, introduced into the recesses 33 can directly contact the absorbent layer 4 at the compressed portions 22c. On the other hand, the absorbing sheet 1 has an advantage in that the color of body fluid absorbed by the absorbent layer 4 can be easily concealed with the top layer 5 because the bottom portion 21b of each recess 21 remains covered with the top layer 5.

The second embodiment of Fig. 6 may be modified such that the fibers constituting the absorbent layer 4 are absent from the bottom portions of the recesses 33 or through-holes are formed in the absorbent layer 4 at the bottom portions of the recesses 33.

The preferred structures of the absorbent layer 4 and the top layer 5 have been already described hereinabove, but the absorbent layer 4 and the top layer 5 may have other structures for the absorbing sheet 1 of Fig. 1 and the absorbing sheet 31 of Fig. 6.

For the absorbent layer 4, use can be made of nonwoven fabric of which constituent fibers are bonded together only with an adhesive. In this case, for instance, the absorbent layer 4 may be air-laid nonwoven fabric only of hydrophilic fibers such as natural fibers (e.g., pulp) and regenerated cellulose fibers (e.g., rayon) or air-laid nonwoven fabric of a mixture of the hydrophilic fibers and synthetic fibers such as the sheath/core bicomponent fibers, wherein the fibers are bonded together by spraying an emulsion type adhesive on both surfaces of the air-laid nonwoven fabric. Also in this case, where the shape of the absorbent layer 4 is maintained with the adhesive, the absorbent layer 4 preferably has a wet tensile strength of at least 2 N per 25 mm width so that the shape of recesses formed therein can be certainly maintained to prevent disappearance of the recesses.

For the top layer 5, other kinds of nonwoven fabric such as spunlaced nonwoven fabric and point-bonded nonwoven fabric may be used in place of the through-air bonded nonwoven fabric. These kinds of nonwoven fabric may have liquid passage holes that have a smaller opening area than the recesses. The nonwoven fabric may be formed of a mixture of synthetic fibers and hydrophilic fibers such as rayon, but it is preferably formed only of synthetic fibers so as to prevent residual liquid in the top layer 5 and also prevent body fluid once retained in the absorbent layer 4 from flowing back to the top layer 5. It is also possible to use spunlaced nonwoven fabric only of hydrophilic fibers for the top layer 5.

In the present invention, as has been described hereinabove, since fibers constituting the absorbent layer 4 are bonded to the top layer 5 around the recesses, the shape of recesses can be certainly maintained. In the case where heat-fusible synthetic fibers are contained in at least one of the top layer 5 and the absorbent layer 4, the top layer 5 and the absorbent layer 4 can be fusion-bonded to each other around the recesses by heat-embossing. Thus, the fused regions can be formed as shown in Fig. 3 or Fig. 6. Likewise, the shape of the recesses can be certainly maintained by bonding the top layer 5 to the absorbent layer 4 at the recesses with a hot-melt type adhesive. At this time, bonding between the top layer 5 and the absorbent layer 4 around the recesses may be performed with the adhesive only or in combination with fusion-bonding.

Next, an exemplary method for manufacturing the absorbing sheet 1 of Fig. 1 will be described with reference to Fig. 7. It should be noted that the absorbing sheet 31 of Fig. 6 can be manufactured in a similar way.

A material roll 41 is the first nonwoven fabric 5a that is wound into the form of a roll; and a material roll 42 is the second nonwoven fabric 5b that is wound into the form of a roll. When wound into the material rolls 41 and 42, both the first nonwoven fabric 5a and the second nonwoven fabric 5b are reduced in thickness.

After passing over a roller 43, the first nonwoven fabric 5a is fed to a heating unit 50a, whereas after passing over a roller 44, the second nonwoven fabric 5b is fed to a heating unit 50b. The heating unit 50a has heating rollers 51a and 52a whose surfaces are heated, and the first nonwoven fabric 5a is wound around the heating rollers 51a and 52a for heating. Likewise, the heating unit 50b has heating rollers 51b and 52b whose surfaces are heated, and the second nonwoven fabric 5b is wound around the heating rollers 51b and 52b for heating.

The heating rollers 51a and 52a are spaced apart from each other and their surfaces are smooth. The first nonwoven fabric 5a is heated by the heating rollers 51a and 52a, while traveling without being compressed between the heating rollers 51a and 52a. At this time, one surface of the first nonwoven fabric 5a contacts the heated surface of the heating roller 51a, whereas the other surface of the first nonwoven fabric 5a contacts the heated surface of the heating roller 52a. Since the first nonwoven fabric 5a is the through-air bonded nonwoven fabric mainly comprised of thermoplastic resin fibers, when heated by the surfaces of the heating rollers 51a and 52a, the first nonwoven fabric 5a can restore bulk (thickness) in the range of 1.5 to 8 times, as well as its density can be reduced to one-third or less. For instance, the first nonwoven fabric 5a has a thickness of 0.5 mm when wound into the material roll 41; but after heated by the heating rollers 51a and 52a, the thickness is restored to 0.75 to 4 mm. The surface temperature of the heating rollers 51a and 52a is preferably about 115 °C.

Likewise, the second nonwoven fabric 5b is heated in the heating unit 50b.

After thickness restoration, the first nonwoven fabric 5a passes over a roller 45 and is then fed to a pair of cooling devices 53a and 54a. The cooling devices 53a and 54a are arranged to face the garment surface and the body surface of the first nonwoven fabric 5a, respectively. The first nonwoven fabric 5a is rapidly cooled by air blowing out from nozzles of the cooling devices 53a and 54a. When the first nonwoven fabric 5a whose bulk is restored by heating is cooled, the fibers constituting the first nonwoven fabric 5a are immobilized in a short time, so that the first nonwoven fabric 5a can be maintained in the state where the bulk is restored. Accordingly, even if a large tensile force is subsequently given to the traveling first nonwoven fabric 5a, the first nonwoven fabric 5a can be prevented from reducing in bulk.

Likewise, after heated by the heating unit 50b, air blows out from cooling devices 53b and 54b to both the garment surface and the body surface of the second nonwoven fabric 5b, so that the second nonwoven fabric 5b is rapidly cooled. Subsequently, a hot-melt type adhesive is applied to the second nonwoven fabric 5b through a nozzle 55. At this time, the hot-melt type adhesive is applied in a pattern of strips, as shown in Fig. 5A.

Then, the first nonwoven fabric 5a after passing over a roller 47 is held between rollers 48 and 49 together with the second nonwoven fabric 5b, so that the first nonwoven fabric 5a and the second nonwoven fabric 5b are bonded to each other to form the top layer 5. The top layer 5 is then held between a heating roller 57 and a heat-embossing roller 58. The heat-embossing roller 58 contains projections that are arranged in a pattern for forming the round seal 25. Thus, the round seal 25 is formed in the top layer 5.

A material roll 61 is air-laid nonwoven fabric 62 that is wound into the form of a roll while being reduced in thickness. After passing over a roller 63, the air-laid nonwoven fabric 62 for forming the absorbent layer 4 is fed to a heating unit 50c. The heating unit 50c has heating rollers 51c and 52c whose surfaces are heated, and the air-laid nonwoven fabric 62 is wound around the heating rollers 51c and 52c for heating. When heated by the surfaces of the heating rollers 51c and 52c, the air-laid nonwoven fabric 62 can restore bulk (thickness). In the case where the air-laid nonwoven fabric 62 is comprised of a mixture of pulp and heat-fusible thermoplastic resin fibers, the thickness can be restored in the range of about 1.2 to 1.6 times, for instance. More specifically, if the air-laid nonwoven fabric 62 has a thickness of 1.6 mm when wound into the material roll 61, the thickness can be restored to 1.9 to 2.4 mm after fed to the heating rollers 51c and 52c. In order that the air-laid nonwoven fabric can restore bulk, the surface temperature of the heating rollers 51c and 52c is preferably 100 °C or more. After heated by the heating unit 50c, the air-laid nonwoven fabric 62 passes over a roller 64 and is then rapidly cooled by air blowing out from cooling devices 53c and 54c to the garment surface and the body surface thereof. Subsequently, a hot-melt type adhesive is applied to the air-laid nonwoven fabric 62 through a nozzle 65. At this time, the adhesive is applied in a pattern of wavy lines, as shown in Fig. 5B, or a pattern of spiral lines, as shown in Fig. 5C. Then, the air-laid nonwoven fabric 62 is cut to the length L2 of Fig. 1 with a cutter 66. Thus, the absorbent layer 4 is prepared. The absorbent layer 4 is held between rollers 67 and 68 together with the top layer 5, so that the absorbent layer 4 and the top layer 5 are bonded to each other. Here, if the length L2 of the absorbent layer 4 is equal to the length L1 of the absorbing sheet 1, the air-laid nonwoven fabric 62 can be bonded to the top layer 5 without cutting by the cutter 66.

The laminate of the absorbent layer 4 (air-laid nonwoven fabric 62) and the top layer 5 is then fed to a recess-forming part 70. The recess-forming part 70 has a heating roller 71 whose surface is smooth and a heat-embossing roller 72 on whose surface a large number of pins having the shape of a cut-off cone are regularly arranged, wherein the rollers 71 and 72 are opposed to each other. The laminate is fed in between the rollers 71 and 72 so that the absorbent layer 4 (air-laid nonwoven fabric 62) faces the roller 71 and the top layer 5 faces the roller 72. The recesses 21 are formed by the pins of the heat-embossing roller 72 that are heated to a temperature of 60 to 160 °C, wherein the roll pressure is set at 98 to 1470 kPa.

A material roll 75 is liquid-impermeable film 76 that is wound into the form of a roll. The film 76 for forming the backsheet 3 is let out through a roller 77. A material roll 81 is release paper 82 for forming the release sheet 7 that is paper coated with a release resin layer and wound into the form of a roll. A hot-melt type adhesive is applied from a nozzle 83 to the release paper 82 that is let out from the material roll 81, thereby forming the pressure sensitive adhesive layer 6. The release paper 82 and the film 76 are pressed between rollers 84 and 85, so that they are bonded to each other. Furthermore, a hot-melt type adhesive is applied from a nozzle 86 to the body surface of the film 76.

Then, the laminate of the top layer 5 and the absorbent layer 4 is bonded to the film 76 between rollers 87 and 88, thereby forming a laminate of the top layer 5, the absorbent layer 4, the film 76 and the release paper 82.

Finally, the laminate is fed in between cutter rollers 91 and 92 for trimming, so that the absorbing sheet 1 are cut out in the shape of Fig. 1.

### EXAMPLES

Hereinbelow, Examples of the present invention will be described, but the present invention should not be understood as limited to Examples.

Table 1 shows properties and evaluation results for Examples and Comparative Examples that were of a structure similar to that of the absorbing sheet of Fig. 1. These Examples and Comparative Examples were different from each other exclusively in the absorbent layer.

### (1) Top Layer

Through-air bonded nonwoven fabric having a basis weight of 25 g/m² was formed of PE/PET sheath/core bicomponent thermoplastic resin fibers having a fineness of 2.2 dtex and a length of 44 mm, of which the core component of PET contained titanium oxide in an amount of 1.0% by weight of the fiber. This through-air bonded nonwoven fabric was used for the first nonwoven fabric 5a and the second nonwoven fabric 5b.

### (2) Backsheet

Used was liquid-impermeable PE film having a basis weight of 23 g/m².

### (3) Absorbent Layer

### (Comparative Example 1)

Fluff pulp was deposited to have a basis weight of 220 g/m² and a density of 0.053 g/cm³.

### (Comparative Example 2)

Fluff pulp was deposited to have a basis weight of 430 g/m² and a density of 0.084 g/cm³.

### (Comparative Example 3)

Prepared was air-laid nonwoven fabric containing chemical pulp (a length of 5 mm) and PE/PET sheath/core bicomponent thermoplastic resin fibers (a fineness of 3.3 dtex; a length of 5 mm) in a weight ratio of 80:20, wherein an emulsion adhesive containing acrylic acid or acrylic derivative was sprayed on both surfaces of the fiber web in an amount of 10% by weight of the fiber web. A single sheet of the air-laid nonwoven fabric was used for the absorbent layer. The absorbent layer had a basis weight of 40 g/m² and a density of 0.038 g/cm³.

### (Example 1)

Two sheets of air-laid nonwoven fabric identical to that used for Comparative Example 3 were stacked one on another and used for the absorbent layer.

### (Example 2)

Prepared was air-laid nonwoven fabric containing chemical pulp (a length of 5 mm) and PE/PET sheath/core bicomponent thermoplastic resin fibers (a fineness of 3.3 dtex; a length of 5 mm) in a weight ratio of 70:30, wherein an emulsion adhesive containing acrylic acid or acrylic derivative was sprayed on both surfaces of the fiber web in an amount of 10% by weight of the fiber web. A single sheet of the air-laid nonwoven fabric was used for the absorbent layer. The absorbent layer had a basis weight of 40 g/m² and a density of 0.035 g/cm³.

### (Example 3)

Prepared was air-laid nonwoven fabric containing chemical pulp (a length of 5 mm) and PE/PET sheath/core bicomponent thermoplastic resin fibers (a fineness of 3.3 dtex; a length of 5 mm) in a weight ratio of 70:30, wherein an emulsion adhesive containing acrylic acid or acrylic derivative was sprayed on both surfaces of the fiber web in an amount of 10% by weight of the fiber web. A single sheet of the air-laid nonwoven fabric was used for the absorbent layer. The absorbent layer had a basis weight of 60 g/m² and a density of 0.053 g/cm³.

### (Comparative Example 4)

Prepared was air-laid nonwoven fabric containing chemical pulp (a length of 5 mm) and PE/PET sheath/core bicomponent thermoplastic resin fibers (a fineness of 3.3 dtex; a length of 5 mm) in a weight ratio of 95:5, wherein an emulsion adhesive containing acrylic acid or acrylic derivative was sprayed on both surfaces of the fiber web in an amount of 10% by weight of the fiber web. A single sheet of the air-laid nonwoven fabric was used for the absorbent layer. The absorbent layer had a basis weight of 100 g/m² and a density of 0.077 g/cm³.

### (Example 4)

Prepared was air-laid nonwoven fabric containing fluff pulp and PE/PP sheath/core bicomponent thermoplastic resin fibers (a fineness of 1.7 dtex; a length of 13 mm) in a weight ratio of 87:13, without using any adhesive. A single sheet of the air-laid nonwoven fabric was used for the absorbent layer. The absorbent layer had a basis weight of 50 g/m² and a density of 0.073 g/cm³.

### (Example 5)

Prepared was air-laid nonwoven fabric containing fluff pulp and PE/PP sheath/core bicomponent thermoplastic resin fibers (a fineness of 1.7 dtex; a length of 13 mm) in a weight ratio of 87:13, without using any adhesive. A single sheet of the air-laid nonwoven fabric was used for the absorbent layer. The absorbent layer had a basis weight of 160 g/m² and a density of 0.071 g/cm³.

In the foregoing Examples and Comparative Examples, the absorbent layer had a width of 30 mm and a length of 100 mm.

### (4) Structure of Absorbing Sheet

The top layer was prepared by bonding two sheets of the through-air bonded nonwoven fabric to each other with a hot-melt type adhesive. This adhesive was applied in a pattern of strips (see Fig. 5A), which were spaced 1 mm apart from each other. The adhesive had a basis weight of 10 g/m².

The top layer and the absorbent layer were bonded to each other with a hot-melt type adhesive. This adhesive was applied in a pattern of a plurality of spiral lines (see Fig. 5C), wherein each spiral has a width of 5 mm. The adhesive had a basis weight of 10 g/m².

As means for forming the recesses, used were a smooth-surface roller whose surface temperature was 130 °C and an embossing roller having pins whose temperature was 125 °C, wherein the roll pressure was set at 1470 kPa and the pressing time of the pins was set at 2 seconds. The pins were of the shape of a cut-off cone, wherein the top diameter was 1 mm, the height was 2 mm, and the taper angle of the cone was 60 degrees. The recesses were arranged such that the longitudinal pitch Py was 5 mm and the transverse pitch Px was 2.5 mm.

The backsheet was bonded to the laminate of the top layer and the absorbent layer with a hot-melt type adhesive. The application pattern and the application amount of this adhesive were identical to those of the adhesive used for bonding two sheets of the through-air bonded nonwoven fabric.

### (5) Measurement of Density

The sheet materials for the absorbent layer were cut into a size of 50 x 50 mm. The sheet material thus cut was held between two acrylic plates, and thickness of the sheet material was measured under a pressure of 392 Pa. Density was calculated from the thickness and the basis weight.

### (6) Wet Strength

The sheet materials for the absorbent layer were cut into a size of 25 x 50 mm. The sheet material thus cut was left for two hours in an atmosphere having a temperature of 20 °C and a relative humidity of 65%. Thereafter, this sample was completely immersed in distilled water, and then quickly taken out. 30 seconds after the sample was taken out, tensile test was performed using a tensile tester " instron 5564" to measure maximum load, wherein chuck distance was set at 25 mm, longitudinally opposed ends of the sample were held with chucks, and the chucks were pulled at a rate of 100 mm/min. The maximum load was taken as wet strength.

### (7) Product Evaluation

0.5 cc of distilled water was dropped onto the liquid absorbing region having the recesses and left for 15 minutes. Thereafter, the surface of the liquid absorbing region was rubbed 100 times under a load of 1.96 N using a rubbing-fastness tester, and then the surface was observed. In Table 1: " ⓞ" indicates the product in which disappearance of the recesses was not observed at all; " ○" indicates the product in which disappearance of the recesses was hardly observed; " Δ" indicates the product in which disappearance of the recesses was observed; and " ×" indicates the product in which the recesses completely disappeared.

**Table 1**

| | Basis Weight (g/m²) | Density (g/cm³) | Wet Strength (N) | Product Evaluation |
|---|---|---|---|---|
| Com. Ex. 1 | 220 | 0.053 | 1.47 | × |
| Com. Ex. 2 | 430 | 0.084 | 1.73 | × |
| Com. Ex. 3 | 40 | 0.038 | 1.29 | Δ |
| Ex. 1 | 80 | 0.038 | 2.45 | ○ |
| Ex. 2 | 40 | 0.035 | 2.06 | ○ |
| Ex. 3 | 60 | 0.053 | 2.81 | ○ |
| Com. Ex. 4 | 100 | 0.077 | 1.62 | Δ |
| Ex. 4 | 50 | 0.073 | 4.26 | ○ |
| Ex. 5 | 160 | 0.071 | 6.20 | ⓞ |

From Table 1, it is seen that in the case where the wet strength of the absorbent layer is equal to or greater than 2 N, the recesses hardly disappear. It is also seen that in the case where the wet strength is equal to or greater than 2 N, the density can be set within the preferred range of 0.035 to 0.13 cm³/g.

In the present invention, as has been described hereinabove, even when a thin absorbent layer is used in the absorbent article, the recesses formed from the top layer to the absorbent layer hardly disappear in a wet state. Accordingly, body fluid can be easily introduced into the absorbent layer through the recesses at all times. In addition, since a relatively bulky sheet can be used for the top layer, the absorbent article can provide a soft feel.

Although the present invention has been illustrated and described with respect to exemplary embodiment thereof, it should be understood by those skilled in the art that the foregoing and various other changes, omission and additions may be made therein and thereto, without departing from the spirit and scope of the present invention. Therefore, the present invention should not be understood as limited to the specific embodiment set out above but to include all possible embodiments which can be embodied within a scope encompassed and equivalent thereof with respect to the feature set out in the appended claims.

## Claims

1. An absorbent article comprising:
a liquid-permeable top layer;
a backsheet; and
an absorbent layer disposed between the top layer and the backsheet, fibers constituting the absorbent layer being bonded together, wherein
the absorbent layer comprises hydrophilic fibers, and
the top layer and the absorbent layer are embossed together to have recesses in which fibers constituting the absorbent layer are bonded to the top layer.

2. An absorbent article as set forth in claim 1, wherein the absorbent layer has a wet tensile strength of at least 2 N per 25 mm width.

3. An absorbent article as set forth in claim 1, wherein the absorbent layer further comprises heat-fusible synthetic fibers, and the synthetic fibers are fusion-bonded to the hydrophilic fibers inside the absorbent layer.

4. An absorbent article as set forth in claim 1, wherein fibers constituting the absorbent layer are bonded together with an adhesive.

5. An absorbent article as set forth in claim 1, wherein the top layer comprises a heat-fusible material and fibers constituting the absorbent layer are fusion-bonded to the top layer in the recesses.

6. An absorbent article as set forth in claim 1, wherein the top layer comprises at least one sheet of nonwoven fabric and has a basis weight of 15 to 100 g/m² and a bulk of 0.2 to 1.5 mm.

7. An absorbent article as set forth in claim 1, wherein the top layer comprises two sheets of nonwoven fabric, and the top layer and the absorbent layer are bonded to each other with an adhesive that is applied in a pattern different from that of an adhesive applied for bonding the two sheets of nonwoven fabric to each other.

8. An absorbent article as set forth in claim 1, wherein the absorbent layer is of an elongated shape, and the top layer and the backsheet are bonded to each other outside the absorbent layer to have side regions extending longitudinally of the absorbent layer.

9. An absorbent article as set forth in claim 1, which is to be used as a vaginal discharge absorbing sheet.

10. A method for manufacturing an absorbent article having a liquid-permeable top layer, a backsheet, and an absorbent layer disposed between the top layer and the backsheet, the method comprising:
letting out an absorbent layer from a material roll, wherein fibers constituting the absorbent layer are bonded together and the absorbent layer comprises hydrophilic fibers, and heating the absorbent layer to restore bulk;
bonding a top layer to the absorbent layer;
embossing the top layer and the absorbent layer together to have recesses;
laying a backsheet on a garment surface of the absorbent layer; and
cutting the top layer and the backsheet into the shape of an absorbent article.

11. An absorbent article manufacturing method as set forth in claim 10, which further comprises heating the top layer to restore bulk before bonding the top layer to the absorbent layer.
